(19) Europäisches Patentamt
European Patent Office
Office européen des brevets

(11) EP 4 071 162 A1

(12) EUROPEAN PATENT APPLICATION
published in accordance with Art. 153(4) EPC

(43) Date of publication:
**12.10.2022 Bulletin 2022/41**

(21) Application number: **20895488.3**

(22) Date of filing: **27.11.2020**

(51) International Patent Classification (IPC):
***C07K 5/083*** (2006.01) ***C07K 7/06*** (2006.01)
***C07K 14/00*** (2006.01) ***A61L 31/04*** (2006.01)
***A61L 31/14*** (2006.01)

(52) Cooperative Patent Classification (CPC):
**A61L 31/04; A61L 31/14; C07K 5/0804; C07K 7/06; C07K 14/00**

(86) International application number:
**PCT/JP2020/044316**

(87) International publication number:
**WO 2021/112014 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2019 JP 2019218181**
**21.08.2020 JP 2020140113**

(71) Applicants:
- **Kyoto University**
**Kyoto-shi, Kyoto 606-8501 (JP)**
- **Sanyo Chemical Industries, Ltd.**
**Kyoto-shi, Kyoto 605-0995 (JP)**

(72) Inventors:
- **SATO, Toshihiko**
**Kyoto-shi, Kyoto 606-8501 (JP)**
- **UEDA, Yuichiro**
**Kyoto-shi, Kyoto 606-8501 (JP)**
- **KAWABATA, Shingo**
**Kyoto-shi, Kyoto 605-0995 (JP)**
- **SOMAMOTO, Satoshi**
**Kyoto-shi, Kyoto 605-0995 (JP)**

(74) Representative: **Mewburn Ellis LLP**
**Aurora Building**
**Counterslip**
**Bristol BS1 6BX (GB)**

(54) **BRONCHIAL EMBOLIZATION MATERIAL**

(57) The present invention aims to provide a material for endobronchial occlusion capable of repairing or replacing tissue. The material for endobronchial occlusion of the present invention is a material for endobronchial occlusion containing a protein (A), wherein the protein (A) contains at least one of a polypeptide chain (Y) or a polypeptide chain (Y'), a total number of the polypeptide chain (Y) and the polypeptide chain (Y') in the protein (A) is 1 to 100, the polypeptide chain (Y) is a polypeptide chain consisting of 2 to 200 tandem repeats of at least one amino acid sequence (X) among an amino acid sequence VPGVG (1) represented by SEQ ID NO: 1, an amino acid sequence GVGVP (4) represented by SEQ ID NO: 4, GPP, GAP, and an amino acid sequence GAHGPAGPK (3) represented by SEQ ID NO: 3, the polypeptide chain (Y') is a polypeptide chain in which each of a total of 5% or less of amino acids in the polypeptide chain (Y) is replaced by at least one of a lysine residue or an arginine residue, with a total number of the lysine and arginine residues being 1 to 100, the protein (A) satisfies the following relational expression (1), the material for endobronchial occlusion has a density of 90 to 450 mg/cm$^3$, and the material for endobronchial occlusion satisfies the following relational expression (2) :

0.50 ≤ [Total number of amino acids constituting the amino acid sequence (X) and amino acid sequence (X') in the protein (A)]/[total number of amino acids constituting the protein (A)] ≤ 0.80 (1),

in the relational expression (1), the amino acid sequence (X') is an amino acid sequence in which each of a total of 60% or less of amino acids in the amino acid sequence (X) is replaced by at least one of a lysine residue or an arginine residue;

$$0.01 \leq Q/P \quad (2)$$

in the relational expression (2), P is the weight of a dried product (DP) of the material for endobronchial occlusion subjected to drying at 100°C and 1 atm for three hours and then drying at 40°C and 1 atm for 15 hours; Q is the weight of a dried product (DQ) of the dried product (DP) subjected to immersion in water having a weight 1000 times the P at 25°C and 1 atm for 72 hours, taking out, drying at 100°C and 1 atm for three hours, and then drying at 40°C and 1 atm for 15 hours.

**Description**

TECHNICAL FIELD

The present invention relates to a material for endobronchial occlusion.

BACKGROUND ART

Conventionally known techniques to prevent air leak from a bronchial stump or a lung stump due to lung resection include one that uses a material for endobronchial occlusion to occlude a bronchi (Patent Literature 1).
However, since the material disclosed in Patent Literature 1 is made of silicone, it does not repair or replace the tissue, and the silicone remains in the affected area, which causes various problems (e.g., infection, delayed healing, and need for removal).

CITATION LIST

- Patent Literature

Patent Literature 1: JP 2004-024864 A

SUMMARY OF INVENTION

- Technical Problem

The present invention aims to provide a material for endobronchial occlusion capable of repairing or replacing tissue.

- Solution to Problem

As a result of extensive studies to solve the above issues, the present inventors arrived at the present invention.
Specifically, the present invention is a material for endobronchial occlusion containing a protein (A),

wherein the protein (A) contains at least one of a polypeptide chain (Y) or a polypeptide chain (Y'),
a total number of the polypeptide chain (Y) and the polypeptide chain (Y') in the protein (A) is 1 to 100,
the polypeptide chain (Y) is a polypeptide chain consisting of 2 to 200 tandem repeats of at least one amino acid sequence (X) among an amino acid sequence VPGVG (1) represented by SEQ ID NO: 1, an amino acid sequence GVGVP (4) represented by SEQ ID NO: 4, GPP, GAP, and an amino acid sequence GAHGPAGPK (3) represented by SEQ ID NO: 3,
the polypeptide chain (Y') is a polypeptide chain in which each of a total of 5% or less of amino acids in the polypeptide chain (Y) is replaced by at least one of a lysine residue or an arginine residue, with a total number of the lysine and arginine residues being 1 to 100,
the protein (A) satisfies the following relational expression (1),
the material for endobronchial occlusion has a density of 90 to 450 mg/cm$^3$, and
the material for endobronchial occlusion satisfies the following relational expression (2):

$$0.50 \leq [\text{Total number of amino acids constituting the amino acid sequence (X) and amino acid sequence (X') in the protein (A)}]/[\text{total number of amino acids constituting the protein (A)}] \leq 0.80 \quad (1),$$

in the relational expression (1), the amino acid sequence (X') is an amino acid sequence in which each of a total of 60% or less of amino acids in the amino acid sequence (X) is replaced by at least one of a lysine residue or an arginine residue;

$$0.01 \leq Q/P \quad (2)$$

in the relational expression (2), P is the weight of a dried product (DP) of the material for endobronchial occlusion subjected to drying at 100°C and 1 atm for three hours and then drying at 40°C and 1 atm for 15 hours; Q is the weight of a dried product (DQ) of the dried product (DP) subjected to immersion in water having a weight 1000 times the P at 25°C and 1 atm for 72 hours, taking out, drying at 100°C and 1 atm for three hours, and then drying at 40°C and 1 atm for 15 hours.

- Advantageous Effects of Invention

The material for endobronchial occlusion of the present invention can be used as a material for endobronchial occlusion to treat air leak from a bronchial stump or a lung stump due to lung resection and promotes tissue repair or replacement.

DESCRIPTION OF EMBODIMENTS

The material for endobronchial occlusion in the present invention is a material for endobronchial occlusion containing a protein (A).

The protein (A) contains at least one of a polypeptide chain (Y) or a polypeptide chain (Y'),

a total number of the polypeptide chain (Y) and the polypeptide chain (Y') in the protein (A) is 1 to 100,
the polypeptide chain (Y) is a polypeptide chain consisting of 2 to 200 tandem repeats of at least one amino acid sequence (X) among an amino acid sequence VPGVG (1) represented by SEQ ID NO: 1, an amino acid sequence GVGVP (4) represented by SEQ ID NO: 4, GPP, GAP, and an amino acid sequence GAHGPAGPK (3) represented by SEQ ID NO: 3,
the polypeptide chain (Y') is a polypeptide chain in which each of a total of 5% or less of amino acids in the polypeptide chain (Y) is replaced by at least one of a lysine residue or an arginine residue, with a total number of the lysine and arginine residues being 1 to 100,
the protein (A) satisfies the following relational expression (1):

$$0.50 \leq [\text{Total number of amino acids constituting the amino acid sequence (X) and amino acid sequence (X') in the protein (A)}]/[\text{total number of amino acids constituting the protein (A)}] \leq 0.80 \quad (1),$$

in the relational expression (1), the amino acid sequence (X') is an amino acid sequence in which each of a total of 60% or less of amino acids in the amino acid sequence (X) is replaced by at least one of a lysine residue or an arginine residue.

The material for endobronchial occlusion of the present invention contains a protein (A) . The protein (A) has at least one of a polypeptide chain (Y) or a polypeptide chain (Y'). Thus, the material for endobronchial occlusion of the present invention has excellent biocompatibility and adequate elasticity adaptable to expansion and contraction of the lungs.

The polypeptide chain (Y) may consist of one or more types of amino acid sequences (X).

Preferred amino acid sequences (X) are VPGVG (1) and GVGVP (4) in terms of biocompatibility and elasticity of the material for endobronchial occlusion and regeneration potential of bronchi and lung tissue.

Specific examples of the polypeptide chain (Y) include $(VPGVG)_b$, $(GVGVP)_c$, and $(GAHGPAGPK)_d$. Each of "b", "c", and "d" represents the number of tandem repeats of the corresponding amino acid sequence (X) and is an integer of 2 to 200.

When there are multiple polypeptide chains (Y) in one molecule of the protein (A), these polypeptide chains (Y) may be the same as or different from each other, and the protein (A) may contain at least one or more selected from the group consisting of $(VPGVG)_b$, $(GVGVP)_c$, and $(GAHGPAGPK)_d$.

In addition, when the protein (A) contains multiple polypeptide chains (Y), the number of repeats of the amino acid sequence (X) may be the same or different for each polypeptide chain (Y). In other words, the protein (A) may contain multiple polypeptide chains (Y) in which the numbers b to d of tandem repeats of the amino acid sequence (X) are the same or different.

The polypeptide chain (Y) is preferably $(VPGVG)_b$ or $(GVGVP)_c$ in terms of biocompatibility and elasticity of the material for endobronchial occlusion.

The polypeptide chain (Y) is a polypeptide chain consisting of 2 to 200 tandem repeats (each of the b to d is 2 to 200) of the amino acid sequence (X). In terms of biocompatibility and elasticity of the material for endobronchial occlusion, the number of tandem repeats of the amino acid sequence (X) is preferably 2 to 100 (each of the b to d is 2 to 100), more preferably 2 to 50 (each of the b to d is 2 to 50), particularly preferably 2 to 40 (each of the b to d is 2 to 40).

The polypeptide chain (Y') is a polypeptide chain in which each of a total of 5% or less of amino acids in the polypeptide chain (Y) is replaced by at least one of a lysine residue or an arginine residue, with a total number of the lysine and arginine residues that replaced being 1 to 100.

The polypeptide chain (Y') can be identified based on whether the polypeptide chain (Y) is obtained when all the lysine (K) and arginine (R) residues in the sequence of the protein (A) are replaced by other amino acids (glycine (G), alanine (A), valine (V), proline (P), or histidine (H)).

In the polypeptide chain (Y'), the percentage of the lysine and/or arginine residues that replaced is preferably 0.06% to 5%, more preferably 0.5 to 5%, particularly preferably 1 to 5%, in terms of biocompatibility of the material for endobronchial occlusion.

The polypeptide chain (Y') may contain an amino acid sequence (X') in which each of a total of 60% or less of amino acids in the amino acid sequence (X) is replaced by at least one of a lysine residue or an arginine residue.

Further, the polypeptide chain (Y') may consist of one or more types of amino acid sequences (X) and/or one or more types of amino acid sequences (X').

Specific examples of the amino acid sequence (X') include amino acid sequence GKGVP (7) represented by SEQ ID NO: 7, an amino acid sequence GKGKP (8) represented by SEQ ID NO: 8, an amino acid sequence GKGRP (9) represented by SEQ ID NO: 9, and an amino acid sequence GRGRP (10) represented by SEQ ID NO: 10.

In terms of biocompatibility of the material for endobronchial occlusion, the amino acid sequence (X') is preferably at least one selected from the group consisting of GKGVP (7), GKGKP (8), and GRGRP (10), more preferably GKGVP (7) and GKGKP (8).

The total number of the polypeptide chain (Y) and the polypeptide chain (Y') in one molecule of the protein (A) is 1 to 100. The total number thereof is preferably 1 to 80, more preferably 1 to 60.

The total number of the polypeptide chain (Y) and the polypeptide chain (Y') in one molecule of the protein (A) in the above range is preferred in terms of biocompatibility and elasticity of the material for endobronchial occlusion.

When the protein (A) contains multiple polypeptide chains (Y) different from each other in terms of the type of amino acid sequence (X) and/or the number of tandem repeats of the amino acid sequence (X), each polypeptide chain is counted as one, and the sum of the counts is the number of the polypeptide chains (Y). The same applies to the polypeptide chain (Y').

As described above, the protein (A) in the present invention is required to satisfy the relational expression (1) .

$$0.50 \leq \text{[Total number of amino acids constituting the amino acid sequence (X) and amino acid sequence (X') in the protein (A)]/[total number of amino acids constituting the protein (A)]} \leq 0.80 \quad (1)$$

A ratio of the number of amino acids of less than 0.50 results in poor regeneration potential of bronchi and lung tissue, and a ratio of more than 0.80 results in poor water solubility.

The ratio of the number of amino acids is preferably 0.50 to 0.70, more preferably 0.525 to 0.675, particularly preferably 0.525 to 0.65, in terms of increase in regeneration potential of bronchi and lung tissue.

The ratio of the number of amino acids can be determined with a protein sequencer. Specifically, the percentage can be determined by the following measurement method.

<Measurement method>

The protein (A) is divided into fragments of about 30 residues or less using at least two methods of cleaving the sequence at a specific amino acid residue. Subsequently, the fragments are separated by high performance liquid chromatography (HPLC), and then the amino acid sequence is analyzed with a protein sequencer. The entire sequence of the protein (A) is determined by peptide mapping of the amino acid sequences obtained. Subsequently, a calculation is performed to determine "[Total number of amino acids constituting the amino acid sequence (X) and amino acid sequence (X') in the protein (A)]/[total number of amino acids constituting the protein (A)]".

In terms of biocompatibility of the material for endobronchial occlusion and regeneration potential of bronchial and lung tissue, the protein (A) preferably contains a polypeptide chain (S) consisting of 2 to 50 tandem repeats of an amino acid sequence GAGAGS (2) represented by SEQ ID NO: 2.

In the polypeptide chain (S), the number of tandem repeats of GAGAGS (2) is preferably 2 to 40, more preferably 2 to 30, particularly preferably 2 to 10, in terms of biocompatibility of the material for endobronchial occlusion.

In the protein (A), the percentage of the number of amino acids in all repeats of GAGAGS (2) relative to the total number of amino acids in the protein (A), i.e., [{number of repeats of GAGAGS (2) in the protein (A) × 6}/{total number of amino acids in the protein (A)} × 100], is preferably 5 to 50%, more preferably 10 to 47.5%, particularly preferably 20 to 45%, in terms of increase in regeneration potential of bronchi and lung tissue.

The percentage of the number of amino acids in all repeats of GAGAGS (2) relative to the total number of amino acids in the protein (A) can be determined by a protein sequencer. Specifically, the percentage is determined by the following measurement method.

<Percentage of the number of amino acids in all repeats of GAGAGS (2) relative to the total number of amino acids in the protein (A)>

The protein (A) is divided into fragments of about 30 residues or less using at least two cleaving methods capable of cleaving the sequence at a specific amino acid residue. Subsequently, the fragments are separated by high performance liquid chromatography (HPLC), and then the amino acid sequence is analyzed with a protein sequencer. The entire sequence of the protein (A) is determined by peptide mapping of the amino acid sequences obtained. Subsequently, the percentage of the number of amino acids in all repeats of GAGAGS (2) relative to the total number of amino acids in the protein (A) is calculated by the following equation:

Percentage (%) of the number of amino acids in all repeats GAGAGS (2) to the total number of amino acids in the protein (A) = [{number of repeats of GAGAGS (2) × 6}/{total number of amino acids in the protein (A)}] × 100.

When the protein (A) contains a total of two or more polypeptide chains of at least one type selected from the group consisting of the polypeptide chain (Y), the polypeptide chain (Y'), and the polypeptide chain (S), an intervening amino acid sequence (Z) may be present between each sequence. The intervening amino acid sequence (Z) is a peptide sequence containing one or more amino acids linked together, and it is none of GAGAGS (2), the amino acid sequence (X), and the amino acid sequence (X'). The number of amino acids constituting the intervening amino acid sequence (Z) is preferably 1 to 30, more preferably 1 to 15, particularly preferably 1 to 10, in terms of biocompatibility and elasticity of the material for endobronchial occlusion. Specific examples of the intervening amino acid sequence (Z) include an amino acid sequence VAAGY (11) represented by SEQ ID NO: 11, an amino acid sequence GAAGY (12) represented by SEQ ID NO: 12, and LGP sequence.

The percentage of the number of amino acids in all intervening amino acid sequences (Z) relative to the total amino acids in the protein (A) [Σ{(number of amino acids in the intervening amino acid sequences (Z)) × (number of intervening amino acid sequences (Z))}/{total number of amino acids in the protein (A)} × 100] is preferably 0 to 25%, more preferably 0 to 22.5%, particularly preferably 0.01 to 15%, in terms of biocompatibility and elasticity of the material for endobronchial occlusion.

In terms of biodegradability in vivo, the protein (A) may contain a terminal amino acid sequence (T) at an end, in addition to GAGAGS (2), the amino acid sequence (X), the amino acid sequence (X'), and the intervening amino acid sequence (Z). The terminal amino acid sequence (T) may be located at one or each end of the protein (A).

The terminal amino acid sequence (T) does not include any of purification tags described below.

A preferred terminal structure of the protein (A) is one in which the terminal amino acid sequence (T) is linked to the polypeptide chain (Y). The terminal amino acid sequence (T) is a peptide sequence containing one or more amino acids linked together, and it is none of the sequence GAGAGS (2), the amino acid sequence (X), and the amino acid sequence (X'). The number of amino acids constituting the terminal amino acid sequence (T) is preferably 1 to 100, more preferably 1 to 50, particularly preferably 1 to 40, in terms of biodegradability in vivo. Specific examples of the terminal amino acid sequence (T) include an amino acid sequence MDPWLQRRDWENPGVTQLNRLAAHPPFASDPM (13) represented by SEQ ID NO: 13.

The percentage of the number of amino acids in the terminal amino acid sequence (T) relative to the total number of amino acids in the protein (A) is preferably 0 to 25%, more preferably 0 to 22.5%, particularly preferably 0.01 to 15%, in terms of biodegradability in vivo.

The protein (A) may be prepared from bacteria using biotechnological techniques as described below. In such a case, the protein (A) may contain, in addition to the terminal amino acid sequence (T), a particular amino acid sequence (hereinafter referred to as "purification tag") at the N or C terminal of the protein (A) in order to facilitate purification or detection of the protein (A) expressed. An affinity purification tag is used as the purification tag. Examples of the purification tag include 6×His tag consisting of polyhistidine, V5 tag, Xpress tag, AU1 tag, T7 tag, VSV-G tag, DDDDK tag, S tag, CruzTag09™, CruzTag22™, CruzTag41™, Glu-Glu tag, Ha.11 tag, and KT3 tag.

Examples of combinations of a purification tag (i) and a ligand (ii) capable of recognizing and binding to the tag are listed below.

(i-1) Glutathione-S-transferase (GTS), (ii-1) Glutathione
(i-2) Maltose binding protein (MBP), (ii-2) Amylose
(i-3) HQ tag, (ii-3) Nickel
(i-4) Myc tag, (ii-4) Anti-Myc antibody
(i-5) HA tag, (ii-5) Anti-HA antibody
(i-6) FLAG tag, (ii-6) Anti-FLAG antibody
(i-7) 6×His tag (ii-7) Nickel or cobalt

The purification tag sequence can be added by, for example, incorporating a nucleic acid encoding the purification tag into the 5' or 3' end of the nucleic acid encoding the protein (A) in an expression vector, or using a commercial vector designed to add the purification tag.

In the protein (A), the percentage of the sum of the number of amino acids in all intervening amino acid sequences (Z) constituting the protein (A), the number of amino acids in all terminal amino acid sequences (T) constituting the protein (A), and the number of amino acids in purification tags is preferably 0 to 25%, more preferably 0 to 22.5%, particularly preferably 0.01 to 15% based on the total number of amino acids in the protein (A), in terms of biodegradability in vivo.

When the protein (A) contains the polypeptide chain (Y) and/or the polypeptide chain (Y') as well as the polypeptide chain (S), the polypeptide chain (Y) or the polypeptide chain (Y') is preferably alternately linked with polypeptide chain (S) via a chemical bond, in terms of biocompatibility and elasticity of the material for endobronchial occlusion.

The ratio of the number of repeats of GAGAGS (2) to the total number of the amino acid sequence (X) and the amino acid sequence (X'), i.e., GAGAGS (2):total number of amino acid sequence (X) and amino acid sequence (X'), is preferably 1:1.5 to 1:20, more preferably 1:1.5 to 1:6, particularly preferably 1:2 to 1:5, in terms of increase in regeneration potential of bronchi and lung tissue.

Some of preferred examples of the protein (A) are listed below.

(A1)Protein in which the amino acid sequence (X) is GVGVP (4)

(A11) Protein containing a polypeptide chain (Y'1) in which one amino acid in a polypeptide chain (Y1) consisting of 2 to 200 tandem repeats of GVGVP (4) is replaced by a lysine (K) residue

(A11-1) Protein containing the polypeptide chain (Y'1) and a polypeptide chain (S1) consisting of 2 to 200 tandem repeats of GAGAGS (2)

(A11-2): Protein containing an amino acid sequence $(GVGVP)_4GKGVP(GVGVP)_3$ (6) (Y'11) represented by SEQ ID NO: 6 in which one amino acid of a polypeptide chain (Y11) of an amino acid sequence $(GVGVP)_8$ (14) consisting of 8 tandem repeats of GVGVP (4) and represented by SEQ ID NO: 14 is replaced by a lysine (K) residue, and the polypeptide chain (S1) consisting of 2 to 200 tandem repeats of GAGAGS (2)

(A11-2-1): Protein containing an polypeptide chain (S1-1) of an amino acid sequence $(GAGAGS)_4$ (5) consisting of 4 tandem repeats of GAGAGS (2) and represented by SEQ ID NO: 5, and $(GVGVP)_4GKGVP$ $(GVGVP)_3$ (6) represented by SEQ ID NO: 6

Specifically, the following proteins are included:

(i) protein (SELP8K) of an amino acid sequence (16) having a molecular mass of about 80 kDa represented by SEQ ID NO: 16 in which an amino acid sequence $(GAGAGS)_2$ (15) represented by SEQ ID NO: 15 is linked by a chemical bond to a sequence consisting of 12 repeats of $(GAGAGS)_4$ (5) and 13 repeats of $(GVGVP)_4GKGVP(GVGVP)_3$ (6) which are alternately linked by a chemical bond; and

(ii) protein (SELP8K4) of an amino acid sequence having a molecular mass of about 30 kDa represented by SEQ ID NO: 27, consisting of 4 repeats of $(GAGAGS)_4$ (5) and 4 repeats of $(GVGVP)_4GKGVP$ $(GVGVP)_3$ (6) which are alternately linked by a chemical bond.

(A11-2-2): Protein containing a polypeptide chain (S1-2) of an amino acid sequence $(GAGAGS)_2$ (15) consisting of 2 tandem repeats of GAGAGS (2) and represented by SEQ ID NO: 15, and $(GVGVP)_4GKGVP(GVGVP)_3$ (6)

(i) Protein (SELP0K) of an amino acid sequence (17) having a molecular mass of about 82 kDa represented by SEQ ID NO: 17, and having a sequence consisting of 17 repeats of $(GAGAGS)_2$ (15) and 17 repeats of $(GVGVP)_4GKGVP(GVGVP)_3$ (6) which are alternately linked by a chemical bond

(A11-3): Protein containing an amino acid sequence $(GVGVP)_6GKGVP(GVGVP)_5$ (18) (Y'12) represented by SEQ ID NO: 18 in which one amino acid in a polypeptide chain consisting of 12 tandem repeats of GVGVP (4) is replaced by a lysine (K) residue, and the polypeptide chain (S1) consisting of 2 to 200 tandem repeats of GAGAGS (2) (A11-3-1): Protein containing an amino acid sequence $(GAGAGS)_4$ (19) consisting of 4 tandem repeats of GAGAGS (2) and represented by SEQ ID NO: 19, and $(GVGVP)_6GKGVP$ $(GVGVP)_5$ (18)

(i) Protein (SELP8K12) of an amino acid sequence (20) having a molecular mass of about 105 kDa represented by SEQ ID NO: 20 in which $(GAGAGS)_2$ (15) is linked by a chemical bond to a sequence consisting 12 repeats of $(GAGAGS)_4$ (19) and 13 repeats of $(GVGVP)_6GKGVP$ $(GVGVP)_5$ (18) which are alternately linked by a chemical bond

(A2): Protein in which the amino acid sequence (X) is VPGVG (1)

(A21): Protein containing a polypeptide chain (Y2) consisting of 2 to 200 tandem repeats of VPGVG (1), and GAGAGS (2)

(i) Protein (ELP1.1) of an amino acid sequence (26) having a molecular mass of about 200 kDa represented by SEQ ID NO: 26 and having a sequence consisting of 40 repeats of GAGAGS (2), 40 repeats of an amino acid sequence $(VPGVG)_4$ (24) represented by SEQ ID NO: 24, and 40 repeats of an amino acid sequence $(VPGVG)_8$ (25) represented by SEQ ID NO: 25 in which 40 blocks each consisting of $(VPGVG)_4$ (24), GAGAGS (2), and $(VPGVG)_8$ (25) linked in the stated order are linked by a chemical bond

(A3): Protein containing the polypeptide chain (Y1) consisting of 2 to 200 tandem repeats of GVGVP (4) and the polypeptide chain (S1) consisting of 2 to 200 tandem repeats of GAGAGS (2)

Specifically, the following protein is included:

(i) protein (SELP6.1) of an amino acid sequence (23) having a molecular mass of about 110 kDa represented by SEQ ID NO: 23 and having a sequence consisting of 5 repeats of an amino acid sequence $(GAGAGS)_8$ (21) represented by SEQ ID NO: 21 and 5 repeats of an amino acid sequence $(GVGVP)_{40}$ (22) represented by SEQ ID NO: 22 which are alternately linked by a chemical bond.

Preferred of these are the protein (SELP8K) of the sequence (16), the protein (SELP0K) of the sequence (17), the protein (SELP8K12) of the sequence (20), the protein (SELP6.1) of the sequence (23), the protein (ELP1.1) of the sequence (26), and the protein (SELP8K4) of the sequence (27) .

The protein (A) may be a protein containing an amino acid sequence having a homology of 70% or more with the amino acid sequence of the protein (SELP8K) of the sequence (16), the protein (SELP0K) of the sequence (17), the protein (SELP8K12) of the sequence (20), the protein (SELP6.1) of the sequence (23), the protein (ELP1.1) of the sequence (26), or the protein (SELP8K4) of the sequence (27) .

The homology is preferably 80% or more, more preferably 90% or more.

The molecular mass of the protein (A) as determined by SDS polyacrylamide gel electrophoresis (SDS-PAGE) is preferably 15 to 200 kDa, more preferably 30 to 150 kDa, particularly preferably 70 to 120 kDa, in terms of biodegradability in vivo.

In the present invention, the degree of hydrophobicity of the protein (A) is preferably 0.2 to 1.2, more preferably 0.4 to 1.0, particularly preferably 0.42 to 0.80, in terms of increase in regeneration potential of bronchi and lung tissue.

The degree of hydrophobicity of the protein (A) shows the degree of hydrophobicity of the protein (A) molecule. The degree of hydrophobicity can be calculated by substituting the following values into a formula described below: the number ($M\alpha$) of amino acid residues of each type constituting the protein (A) molecule; the degree of hydrophobicity ($N\alpha$) of each amino acid; and the total number (MT) of amino acid residues in one molecule of the protein (A). The degree of hydrophobicity of each amino acid is expressed using the following values described in Non-Patent Literature (Albert L. Lehninger, David L. Nelson, Lehninger Principles of Biochemistry, first volume, Hirokawa Books, September 2010, pp. 346 to 347).

$$\text{Degree of hydrophobicity} = \Sigma(M\alpha \times N\alpha)/(MT)$$

$M\alpha$: number of amino acid residues of each type in one molecule of the artificial protein (A)

$N\alpha$: hydrophobicity of each amino acid

MT: total number of amino acids in one molecule of the artificial protein (A)

A (alanine): 1.8
R (arginine): -4.5
N (asparagine): -3.5
D (aspartic acid): -3.5
C (cysteine): 2.5
Q (glutamine): -3.5
E (glutamic acid): -3.5
G (glycine): -0.4
H (histidine): -3.2
I (isoleucine): 4.5
L (leucine): 3.8
K (lysine): -3.9
M (methionine): 1.9
F (phenylalanine): 2.8
P (proline): -1.6
S (serine): -0.8
T (threonine): -0.7
W (tryptophan): -0.9
Y (tyrosine): -1.3
V (valine): 4.2

For example, when the artificial protein (A) is $(GVGVP)_4GKGVP(GVGVP)_3$ (6), the degree of hydrophobicity of the artificial protein (A) = {16 (number of G) × (-0.4) + 15 (number of V) × 4.2 + 8 (number of P) × (-1.6) + 1 (number of K) × (-3.9)}/40 (total number of amino acids) = 1.0.

Since the material for endobronchial occlusion of the present invention contains the protein (A) consisting of the amino acid sequences described above, it is decomposed by enzymes in the body, thus exhibiting excellent biodegradability.

In the present invention, the protein (A) can be obtained by the following methods: extraction from natural products, organic synthesis (e.g. enzymatic synthesis, solid-phase peptide synthesis, and liquid-phase synthesis), genetic recombination, and the like. Examples of the organic synthesis include methods described in "Seikagaku Jikken Koza 1 (Biochemistry Experimental Course 1), Chemistry of Protein IV (edited by Japan Society for Biochemistry, published by Tokyo Kagakudojin on July 1, 1981)" and "*Zoku Seikagaku Jikken Koza* 2) (Sequel to Biochemistry Experimental Course 2, Chemistry of Protein (Vol. 2) (edited by Japan Society for Biochemistry, published by Tokyo Kagakudojin on May 20, 1987)". Examples of the genetic recombination include the method disclosed in Japanese Patent No. 3338441. The protein (A) can be obtained by extraction from natural products, organic synthesis, or genetic recombination. Yet, the genetic recombination is preferred in terms of easy alteration of amino acid sequences and mass productivity at low cost.

The material for endobronchial occlusion of the present invention has a density of 90 to 450 mg/cm$^3$. Preferably, the material for endobronchial occlusion of the present invention is spongy.

A density of the material for endobronchial occlusion of less than 90 mg/cm$^3$ results in poor endobronchial occlusion effect. A density of more than 450 mg/cm$^3$ results in poor repair of bronchi and lung tissue.

The density of the material for endobronchial occlusion is preferably 90 to 300 mg/cm$^3$, more preferably 95 to 250 mg/cm$^3$, still more preferably 100 to 225 mg/cm$^3$, in terms of endobronchial occlusion effect and repair of bronchi and lung tissue.

The density can be set within the above range by, for example, a method that adjusts the concentration of an aqueous solution containing the protein (A) within a preferred range (described later) in a lyophilization step in a method of molding the material for endobronchial occlusion (described in detail later).

The material for endobronchial occlusion of the present invention is required to satisfy the following relational expression (2).

$$0.01 \leq Q/P \quad (2)$$

In the relational expression (2), P is the weight of a dried product (DP) of the material for endobronchial occlusion subjected to drying at 100°C and 1 atm for three hours and then drying at 40°C and 1 atm for 15 hours.

In the relational expression (2), Q is the weight of a dried product (DQ) of the dried product (DP) subjected to immersion in water having a weight 1000 times the P at 25°C and 1 atm for 72 hours, taking out, drying at 100°C and 1 atm for three hours, and then drying at 40°C and 1 atm for 15 hours.

A value of Q/P of less than 0.01 results in a poor bronchi and lung tissue repairing effect.

The Q/P is preferably 0.01 to 0.90 in terms of endobronchial occlusion effect and repair of bronchi and lung tissue.

To set the Q/P within the above range, it is preferred that an organic solvent treatment step is performed in the method of molding the material for endobronchial occlusion (described in detail later).

Also, the Q/P can be set within the above range by setting the degree of hydrophobicity of the protein (A) within the preferred range described above.

Preferably, the material for endobronchial occlusion of the present invention further satisfies the following relational expression (3).

$$0.3 \leq R/P \quad (3)$$

In the relational expression (3), P is the weight of a dried product (DP) of the material for endobronchial occlusion subjected to drying at 100°C and 1 atm for three hours and then drying at 40°C and 1 atm for 15 hours.

In the relational expression (3), R is the weight of a dried product (DR) of the dried product (DP) subjected to immersion in water having a weight 1000 times the P at 25°C and 1 atm for two hours, taking out, drying at 100°C and 1 atm for three hours, and then drying at 40°C and 1 atm for 15 hours.

A value of R/P within the above range results in a higher bronchi and lung tissue repairing effect.

The R/P is more preferably 0.3 to 1.00, particularly preferably 0.95 to 1.00, more particularly preferably 0.96 to 1.00, and most preferably 1.00, in terms of endobronchial occlusion effect and repair of bronchi and lung tissue.

To set the R/P within the above range, it is preferred that the organic solvent treatment step is performed in the method of molding the material for endobronchial occlusion (described in detail later).

Preferably, the material for endobronchial occlusion of the present invention is produced by molding the protein (A) by the following method.

A preferred molding method is, for example, a method in which an aqueous solution containing the protein (A) is lyophilized, and then the resulting product is subjected to treatment with an organic solvent (sometimes abbreviated as "organic solvent treatment").

The following describes the lyophilization step and the organic solvent treatment step.

Preferably, in the lyophilization step, an aqueous solution containing the protein (A) is charged into a frame (e.g., a mold) having a desired mold shape, and lyophilized.

The weight percent of the polypeptide (A) contained in the aqueous solution is preferably 12.5 to 150 g/L, more preferably 25 to 100 g/L, particularly preferably 25 to 50 g/L, based on the volume of the aqueous solution, in terms of adjustment of the density of the material for endobronchial occlusion within the range described above.

Preferably, the lyophilization includes primary drying and secondary drying.

The temperature during the primary drying is preferably -35°C to -5°C in terms of moldability.

The degree of vacuum during the primary drying is preferably 0 to 20 Pa in terms of moldability.

The duration of the primary drying is preferably 10 to 100 hours in terms of moldability.

The temperature during the secondary drying is preferably 5°C to 35°C, more preferably 5°C to 30°C, in terms of moldability.

The degree of vacuum during the secondary drying is preferably 0 to 20 Pa in terms of moldability.

The duration of the secondary drying is preferably 10 to 100 hours in terms of moldability.

In the organic solvent treatment step, preferably, the dried product obtained in the lyophilization step is immersed in an organic solvent (preferably immersed at 1°C to 40°C for 1 to 168 hours), and subsequently, the organic solvent is heated (preferably at 5°C to 30°C and a relative humidity of 10 to 80% RH) for removal.

An immersion time of one hour or more is preferred because the value of "Q/P" can be adjusted within the above range. An immersion time of 168 hours or less is preferred in terms of preservation of the protein (A).

In the organic solvent treatment step, the amount of the organic solvent is preferably an amount that allows the dried product obtained in the lyophilization step to be entirely immersed.

The organic solvent is not limited, and may be a hydrophobic organic solvent such as hexane or a hydrophilic organic solvent.

Preferably, the SP value and/or HLB value of the hydrophilic organic solvent are/is in a preferred range described below. Specific examples of the hydrophilic organic solvent include C1-C4 alcohols (e.g., methanol, ethanol, propanol, and butanol), C3-C6 ketones (e.g., acetone, ethyl methyl ketone, and methyl isobutyl ketone), C2-C6 glycols (e.g., ethylene glycol, propylene glycol, and diethylene glycol) and their monoalkyl (C1-C2) ethers, dimethylformamide, and C3-C5 alkyl acetates (e.g., methyl acetate and ethyl acetate).

Preferred of these organic solvents are C1-C4 alcohols, and more preferred are methanol and ethanol, in terms of moldability of the material for endobronchial occlusion, endobronchial occlusion effect, and repair of bronchi and lung tissue.

The SP value of the organic solvent is preferably 5 to 20 $(cal/cm^3)^{1/2}$, more preferably 12 to 20 $(cal/cm^3)^{1/2}$, particularly preferably 13.5 to 15.0 $(cal/cm^3)^{1/2}$, in terms of moldability of the material for endobronchial occlusion, endobronchial occlusion effect, and repair of bronchi and lung tissue.
In the present invention, the SP value is calculated by the method described in Polymer Engineering and Science by Robert F. Fadors et al., vol. 14, pp. 151 to 154.
The HLB value of the organic solvent is preferably 0 to 75, more preferably 20 to 60, particularly preferably 30 to 60, in terms of moldability of the material for endobronchial occlusion, endobronchial occlusion effect, and repair of bronchi and lung tissue.
In the present invention, the HLB value is an indicator that indicates the hydrophilic-lipophilic balance. The HLB value can be calculated from a ratio of the organicity value and the inorganicity value of an organic compound by, for example, the Oda method described in *Kaimen kasseizai nyumon* (Introduction to Surfactants) (Takehiko Fujimoto, published by Sanyo Chemical Industries, Ltd. 2007), p. 212.

$$HLB = 10 \times inorganicity/organicity$$

The organicity and inorganicity values to determine the HLB can be calculated using values listed in the table on page 213 in *Kaimen kasseizai nyumon.*
The material for endobronchial occlusion of the present invention is used for endobronchial occlusion in treatment of a bronchial stump fistula that occurs after lung cancer surgery, refractory pneumothorax, empyema with fistula, and the like.
Specifically, the material for endobronchial occlusion is used in surgeries for bronchial fistula occlusion, greater omentum filling, muscle flap closure, and the like.
In treatments for bronchial fistula occlusion, it is possible to use, for example, a method of inserting and fixing the material for endobronchial occlusion of the present invention in a bronchus causing a fistula by transbronchial approach using a bronchoscope.
In surgeries for greater omentum filling and muscle flap closure, it is possible to use, for example, a method of directly suturing, and inserting and fixing the material for endobronchial occlusion of the present invention, instead of a greater omentum or a muscle flap, in a fistula site by thoracic approach such as RATS, VATS, or thoracotomy.
These methods are methods of the present invention for treating bronchial stump fistula, refractory pneumothorax, and empyema with fistula.
The material for endobronchial occlusion of the present invention has an effect of repairing or replacing tissue. Thus, the material for endobronchial occlusion reduces the incidence of infection and complications and the incidence of repeated pneumothorax after occlusion, compared to conventional materials made of silicone, and is thus useful as a material for treatment.
In addition, use of the material for endobronchial occlusion to occlude a bronchus in at least one disease selected from the group consisting of bronchial stump fistula, refractory pneumothorax, and empyema with fistula is use of the material for endobronchial occlusion of the present invention.

## EXAMPLES

The present invention is more specifically described below with reference to the examples, but the present invention is not limited to these examples.
Hereinafter, "part(s)" means part(s) by weight.

### <Example 1: Production of material for endobronchial occlusion>

### Production of SELP8K

### Production of SELP8K-producing strain

Plasmid pPTS0345 encoding SELP8K was prepared by the method disclosed in EXAMPLES of Japanese Patent No. 4088341.
The plasmid produced was transformed into E. coli cells, thus obtaining a SELP8K-producing strain. The following describes a method of producing SELP8K (polypeptide (A1)) as a type of the polypeptide (A), which is a polypeptide of a sequence (12) having a molecular mass of about 80 kDa and having a sequence consisting of 12 repeats of $(GAGAGS)_4$ (6) and 13 repeats of $(GVGVP)_4GKGVP(GVGVP)_3$ (7) which are alternately linked by a chemical bond.

Culture of SELP8K-producing strain

A culture solution of the SELP8K-producing strain which had been cultured at 30°C overnight was inoculated to 50 ml of LB medium in a 250 ml flask. Kanamycin was added to the LB medium to give a final concentration of 50 μg/ml to obtain a culture solution, and the culture solution was incubated with agitation (200 rpm) at 30°C. When the turbidity (OD 600) of the culture solution reached 0.8 (measured with spectrophotometer UV1700, Shimadzu Corporation), 40 ml of the culture solution was transferred to another flask pre-warmed to 42°C and incubated at 42°C for about two hours. Subsequently, the culture solution was chilled on ice, and the turbidity (OD 600) of the culture solution was measured. E. coli cells were collected by centrifugation.

Purification of SELP8K

The collected E. coli cells were used to purify protein from E. coli biomass by the following steps: step (1): lysis; step (2): removal of insoluble cellular debris by centrifugation; step (3): ammonium sulfate precipitation; step (4): ultrafiltration; step (5): cation exchange chromatography; step (6): ultrafiltration; and step (7): lyophilization. In this manner, purified SELP8K (polypeptide (A1)) having a molecular mass of about 85 kDa was obtained. Subsequently, step 8: molding (lyophilization) and step 9: molding (organic solvent treatment) were performed, whereby a material for endobronchial occlusion was obtained.

Step 1: Lysis

Deionized water (200 g) was added to the collected E. coli cells (100 g), followed by lysis with a high-pressure homogenizer (55 MPa). Thus, a lysate containing lysed cells was obtained. Subsequently, the pH of the lysate was adjusted to 4.0 with glacial acetic acid.

Step 2: Removal of insoluble cellular debris by centrifugation

The lysate was further centrifuged (6300 rpm, 4°C, 30 min) to collect the supernatant.

Step 3: Ammonium sulfate precipitation

A saturated ammonium sulfate solution was added to the supernatant collected in step 2 to give an ammonium sulfate concentration of 25 wt%, followed by standing for 8 to 12 hours. Then, the precipitate was collected by centrifugation. The collected precipitate was dissolved in deionized water. Then, to the solution was added a saturated ammonium sulfate solution to give an ammonium sulfate concentration of 25 wt%, followed by standing for 8 to 12 hours. Then, the precipitate was collected by centrifugation. The collected precipitate was dissolved in deionized water to obtain a solution.

Step 4: Ultrafiltration

The solution obtained in step 3 was applied to an ultrafilter with a cut-off molecular weight of 30,000 (Hollow Fiber, GE Healthcare). The solution obtained in step 3 was ultrafiltered with deionized water in an amount 20 times the volume of the solution. Thus, an ultrafiltered polypeptide solution was obtained.

Step 5: Cation exchange chromatography

The ultrafiltered polypeptide solution was added to 10 mM sodium acetate buffer to give a polypeptide concentration of 20 g/L, and then applied to AKTA Prime (Amersham) to which a cation exchange column (Hi PrepSP XL16/10, GE Healthcare) was connected. Then, 500 mM sodium acetate buffer was used as an eluent, and the eluted fraction was collected.

Step 6: Ultrafiltration

The eluted fraction obtained in step 5 was treated in the same manner as in "4: Ultrafiltration" described above, whereby an ultrafiltered polypeptide solution was obtained.

Step 7: Lyophilization

The polypeptide solution obtained in step 6 was diluted with deionized water to give a polypeptide concentration of 3 g/L, and poured into a stainless steel vat to a water level of not more than 10 mm. Subsequently, the solution was placed in a lyophilizer (Nihon Techno Service Co., Ltd.), and lyophilized at -30°C over 24 hours. The lyophilized product was subjected to primary drying at a vacuum of 5 Pa or less at -30°C over 110 hours, and then to secondary drying at a vacuum of 5 Pa or less at 30°C over 48 hours. Thus, SELP8K (protein (A-1)) was obtained. The protein (A) was identified by Western blotting described below.

In the protein (A-1), the value of "[Total number of amino acids constituting the amino acid sequence (X) and amino acid sequence (X') in the protein (A)]/[total number of amino acids constituting the protein (A)]" is 0.54.

The ratio of the number of repeats of GAGAGS (2) to the total number of the amino acid sequence (X) and the amino acid sequence (X') in one molecule of the protein (A-1), i.e., GAGAGS (2):total number of amino acid sequence (X) and amino acid sequence (X'), is 1:2.

Step 8: Molding (lyophilization)

The protein (A-1) obtained in step 7 was diluted with deionized water to obtain a SELP8K solution (concentration of protein (A-1): 12.5 g/L), and 1 ml of the SELP8K solution was placed in a cylindrical mold having a base area of 1.9 $mm^2$ and a depth of 1.7 mm, followed by lyophilization under the same conditions as in step 7, whereby the SELP8K solution was molded into a spongy form.

Step 9: Molding (organic solvent treatment)

SELP8K molded in step 8 was immersed in methanol (10 mL) and left standing at 4°C for 45 hours. Subsequently, the methanol was completely removed at 25°C and a relative humidity of 30% RH, whereby a material for endobronchial occlusion (Z-1) was obtained.

Identification of SELP8K (A-1)

Analysis was performed by Western blotting using a rabbit anti-SELP8K antibody and a rabbit anti-6×His antibody (Roland Corporation) against 6×His tag at the C terminal. Western Blotting was performed by the following procedures. A band exhibiting reactivity with each antibody was found at an apparent molecular mass of 80 kDa.

Table 1 shows the composition ratio (measured values) of amino acids of the protein (A) obtained by amino acid composition analysis using an amino acid analysis system (Prominence, Shimadzu Corporation), and the composition ratio (theoretical values) of amino acids of SELP8K inferred from the synthetic gene sequence.

The above confirmed that the protein (A) was a protein (SELP8K) containing of a sequence (16) in which an amino acid sequence $(GAGAGS)_2$ (15) represented by SEQ ID NO: 15 is linked by a chemical bond to a sequence consisting of 13 repeats of the polypeptide chain (Y'11) of $(GVGVP)_4GKGVP(GVGVP)_3$ (6) in which one of valine (V) residues in the polypeptide chain (Y) consisting of 8 tandem repeats of GVGVP (4) is replaced by a lysine (K) residue, and 12 repeats of the polypeptide chain (S1-1) of $(GAGAGS)_4$ (5) consisting of 4 tandem repeats of GAGAGS (2), with these sequences being alternately linked by a chemical bond.

[Table 1]

| Amino acid | Measured value | Theoretical value |
|---|---|---|
| | Composition ratio (%) | Composition ratio (%) |
| Ala | 12.3 | 12.2 |
| Asx | 0.9 | 0.8 |
| Glx | n.d. | 0.4 |
| Phe | 0.4 | 0.1 |
| Gly | 43.7 | 41.5 |
| His | 0.4 | 0.8 |
| Ile | 0.3 | 0 |
| Lys | 1.5 | 1.5 |
| Leu | 0.3 | 0.5 |
| Met | 0.3 | 0.3 |

(continued)

| Amino acid | Measured value | Theoretical value |
|---|---|---|
| | Composition ratio (%) | Composition ratio (%) |
| Pro | 11.7 | 12.4 |
| Arg | 0.5 | 0.6 |
| Ser | 5.3 | 6.1 |
| Thr | n.d. | 0.1 |
| Val | 21.2 | 22.4 |
| Tyr | 1.1 | 0.1 |

<Western blotting>

A sample for Western blotting (20 μL) was mixed with 10 μL of 3× SDS treatment buffer (150 mM Tris HCl (pH 6.8), 300 mM dithiothreitol, 6 wt% dodecyl sodium sulfate (SDS), 0.3 wt% bromophenol blue, and 30 wt% glycerol), and heated at 95°C for 5 minutes. Thus, a sample for electrophoresis was prepared. SDS-PAGE was carried out with the sample for electrophoresis (15 μL) . After electrophoresis, the gel was transferred to a polyvinylidene fluoride membrane (hereinafter abbreviated as "membrane"), and immersed in blocking buffer (20 mM Tris (pH 7.6), 137 mM NaCl, 0.1 wt% Tween 20, and 5 wt% skim milk) with shaking at room temperature for one hour. Thus, the membrane was blocked. After blocking, the membrane was washed with TBS-T (20 mM Tris (pH 7.6), 137 mM NaCl, and 0.1 wt% Tween 20) for two minutes. Next, the membrane was immersed in a solution of primary antibody (a 1:500 dilution of primary antibodies (anti-SELP8K antibody and anti-His-tag antibody (Rockland Immunochemicals Inc.) in TBS-T)), and left standing at 4°C overnight for antibody reaction. After the reaction, the membrane was washed four times in TBS-T (five minutes per time) and immersed in a solution of a secondary antibody capable of binding to the primary antibodies and containing horseradish peroxidase as a marker enzyme (a solution of a secondary antibody is a 1:2000 dilution of a secondary antibody (ECL anti-rabbit IgG HRP linked F(ab')2 fragment (GE Healthcare)) in TBS-T), and left standing at room temperature for 30 minutes for antibody reaction. After the reaction, the membrane was washed four times in TBS-T for (five minutes per time), and enzyme reaction was carried out using ECL-Advance Western Blotting Detection Kit (GE Healthcare). A luminometer For ECL (GE Healthcare) was used to expose the membrane to a high speed black and white instant film (FUJIFILM Corporation) to visualize bands.

<Examples 2 to 6: Production of material for endobronchial occlusion>

Example 1 was repeated except that the concentration of the protein (A-1) in the SELP8K solution in step 8 was changed from 12.5 g/L to 25, 40, 50, 100, or 150 g/L, thus obtaining embolic materials (Z-2) to (Z-6).

<Examples 7 to 9: Production of material for endobronchial occlusion>

Example 4 was repeated except that the methanol for use in step 9 was replaced by hexane, ethanol, or ethylene glycol, thus obtaining embolic materials (Z-7) to (Z-9).

<Example 10: Production of material for endobronchial occlusion>

Example 4 was repeated except that "plasmid pPT0345 encoding SELP8K" was replaced by "plasmid pPT0364 encoding SELP0K", thus obtaining a material for endobronchial occlusion (Z-10) containing a protein (SELP0K) of an amino acid sequence (17) having a molecular mass of about 82 kDa represented by SEQ ID NO: 17 and having a sequence consisting of 17 repeats of $(GAGAGS)_2$ (15) and 17 repeats of $(GVGVP)_4GKGVP(GVGVP)_3$ (6) which are alternately linked by a chemical bond.

<Example 11: Production of material for endobronchial occlusion>

Example 4 was repeated except that "plasmid pPT0345 encoding SELP8K" was replaced by "plasmid pPT0345-4 encoding SELP8K4", thus obtaining a material for endobronchial occlusion (Z-11) containing a protein (SELP8K4) of an amino acid sequence (27) having a molecular mass of about 30 kDa represented by SEQ ID NO: 27 and consisting of 4 repeats of $(GAGAGS)_4$ (5) and 4 repeats of $(GVGVP)_4GKGVP(GVGVP)_3$ (6) which are alternately linked by a chemical bond.

<Example 12: Production of material for endobronchial occlusion>

Example 4 was repeated except that "plasmid pPT0345 encoding SELP8K" was replaced by "plasmid pPT0345-12 encoding SELP8K12", thus obtaining a material for endobronchial occlusion (Z-12) containing a protein (SELP8K12) of an amino acid sequence (20) having a molecular mass of about 105 kDa represented by SEQ ID NO: 20 in which $(GAGAGS)_2$ (15) is linked by a chemical bond to a sequence consisting of 12 repeats of $(GAGAGS)_4$ (19) and 13 repeats of $(GVGVP)_6GKGVP(GVGVP)_5$ (18) which are alternately linked by a chemical bond.

<Example 13: Production of material for endobronchial occlusion>

Example 4 was repeated except that "plasmid pPT0345 encoding SELP8K" was replaced by "plasmid pPT0102-1 encoding ELP1.1", thus obtaining a material for endobronchial occlusion (Z-13) containing a protein (ELP1.1) of an amino acid sequence (26) having a molecular mass of about 200 kDa represented by SEQ ID NO: 26 and having a sequence consisting of 40 repeats of GAGAGS (2), 40 repeats of an amino acid sequence $(VPGVG)_4$ (24) represented by SEQ ID NO: 24, and 40 repeats of an amino acid sequence $(VPGVG)_8$ (25) represented by SEQ ID NO: 25 in which 40 blocks each consisting of $(VPGVG)_4$ (24), GAGAGS (2), and $(VPGVG)_8$ (25) linked in the stated order are linked by a chemical bond.

<Example 14: Production of material for endobronchial occlusion>

Example 4 was repeated except that "plasmid pPT0345 encoding SELP8K" was replaced by "plasmid pPT0270-1 encoding SELP6.1", thus obtaining a material for endobronchial occlusion (Z-14) containing a protein (SELP6.1) of an amino acid sequence (23) having a molecular mass of about 110 kDa represented by SEQ ID NO: 23 and having a sequence consisting of 5 repeats of an amino acid sequence $(GAGAGS)_8$ (21) represented by SEQ ID NO: 21 and 5 repeats of an amino acid sequence $(GVGVP)_{40}$ (22) represented by SEQ ID NO: 22 which are alternately linked by a chemical bond.

<Comparative Examples 1 and 2: Production of material for endobronchial occlusion for comparison>

Example 1 was repeated except that the concentration of the protein (A-1) in the SELP8K solution in step 8 was changed from 12.5 g/L to 10 or 200 g/L, thus obtaining materials for endobronchial occlusion (Z'-1) and (Z'-2) for comparison.

<Comparative Example 3: Production of material for endobronchial occlusion for comparison>

Example 4 was repeated except that "Step 9: Molding (organic solvent treatment)" was not performed. Thus, a molded article obtained in step 8 was used as a material for endobronchial occlusion (Z'-3) for comparison.

The following methods were used to evaluate the density, values of Q/P, and values of R/P of the materials for endobronchial occlusion obtained in Examples 1 to 14 and the materials for endobronchial occlusion for comparison obtained in Comparative Examples 1 to 3. Table 2 shows the results.

<Density>

The density of each material for endobronchial occlusion was calculated from the volume (determined by measuring the base area and height of the obtained cylindrical material for endobronchial occlusion) and the weight of the material for endobronchial occlusion. Table 2 shows the results.

<Q/P>

Using a circulation dryer, the material for endobronchial occlusion was dried at 100°C and 1 atm for three hours and then dried at 40°C and 1 atm for 15 hours, whereby a dried product (DP) was obtained. The weight of the dried product (DP) was denoted by P.

Water having a weight 1000 times the P was added to a plastic cup, and the dried product (DP) was immersed therein and left standing at 25°C and 1 atm for 72 hours. Then, the dried product was taken out.

Using a circulation dryer, the resulting product was dried at 100°C and 1 atm for three hours and then dried at 40°C and 1 atm for 15 hours to obtain a dried product (DQ), and its weight was determined. The weight of the (DQ) was denoted by Q.

The ratio Q/P was calculated using the P and the Q. Table 2 shows the results.

<R/P>

Using a circulation dryer, the material for endobronchial occlusion was dried at 100°C and 1 atm for three hours and then dried at 40°C and 1 atm for 15 hours, whereby a dried product (DP) was obtained. The weight of the dried product (DP) was denoted by P.

Water having a weight 1000 times the P was added to a plastic cup, and the dried product (DP) was immersed therein and left standing at 25°C and 1 atm for two hours. Then, the dried product was taken out.

Using a circulation dryer, the resulting product was dried at 100°C and 1 atm for three hours and then dried at 40°C and 1 atm for 15 hours to obtain a dried product (DR), and its weight was determined. The weight of the (DR) was denoted by R.

The ratio R/P was calculated using the P and the R. Table 2 shows the results.

[Table 2]

| | | Material for endobronchial occlusion | [Total number of amino acids constituting the amino acid sequence (X) and amino acid sequence (X) in the protein (A)]/total number of amino acids constituting the protein (A)] | Degree of hydrophobicity of protein (A) | Concentration of protein (A) in protein (A) solution in step 8 (g/L) | Organic solvent for use in step 9 | Density (mg/cm$^3$) | Q/P | R/P |
|---|---|---|---|---|---|---|---|---|---|
| Example | 1 | Z-1 | 0.54 | 0.62 | 12.5 | Methanol | 90 | 0.47 | 1 |
| | 2 | Z-2 | 0.54 | 0.62 | 25 | Methanol | 100 | 0.99 | 1 |
| | 3 | Z-3 | 0.54 | 0.62 | 40 | Methanol | 150 | 0.90 | 1 |
| | 4 | Z-4 | 0.54 | 0.62 | 50 | Methanol | 200 | 0.71 | 1 |
| | 5 | Z-5 | 0.54 | 0.62 | 100 | Methanol | 250 | 0.84 | 1 |
| | 6 | Z-6 | 0.54 | 0.62 | 150 | Methanol | 300 | 0.72 | 1 |
| | 7 | Z-7 | 0.54 | 0.62 | 50 | Hexane | 200 | 1 | 1 |
| | 8 | Z-8 | 0.54 | 0.62 | 50 | Ethanol | 120 | 0.1 | 0.95 |
| | 9 | Z-9 | 0.54 | 0.62 | 50 | Ethylene glycol | 100 | 0.01 | 0.3 |
| | 10 | Z-10 | 0.72 | 0.72 | 50 | Methanol | 150 | 0.5 | 1 |
| | 11 | Z-11 | 0.52 | 0.72 | 50 | Methanol | 250 | 0.6 | 1 |
| | 12 | Z-12 | 0.69 | 0.84 | 50 | Methanol | 200 | 1 | 1 |
| | 13 | Z-13 | 0.71 | 1.12 | 50 | Methanol | 450 | 1 | 1 |
| | 14 | Z-14 | 0.77 | 1.02 | 50 | Methanol | 400 | 1 | 1 |
| Comparative Example | 1 | Z-1 | 0.54 | 0.62 | 10 | Methanol | 80 | 0.45 | 1 |
| | 2 | Z-2 | 0.54 | 0.62 | 200 | Methanol | 310 | 0.58 | 1 |
| | 3 | Z-3 | 0.54 | 0.62 | 50 | - | 100 | 0.001 | 0.001 |

<Evaluation Examples 1 to 15 and Comparative Evaluation Examples 1 to 3: Evaluation of material for endobronchial occlusion>

An adult beagle dog (1 to 2 years old, weighing 9 to 11 kg) with open chest under general anesthesia underwent lung resection (resection of a site described in Table 3).

A material for endobronchial occlusion disclosed in Table 3 was fixed at four points of a bronchial stump using absorbable sutures, without suture closure to join inner membranes of the bronchial stump with each other. No major air leak was confirmed, and the surgical wound was sutured.

The lumen was bronchoscopically observed two weeks, four weeks, and eight weeks after the surgery to evaluate the degree of absorption of the material for endobronchial occlusion, the degree of occlusion, the presence or absence of displacement, the presence or absence of air leak, and the occurrence of subcutaneous emphysema.

The beagle dog was sacrificed eight weeks after the surgery, and tissues including the bronchi and the bronchial stump with the material for endobronchial occlusion left therein were collected to evaluate the histological degree of endobronchial occlusion, the degree of repair, and the degree of granulation.

Two other adult beagle dogs were also evaluated in terms of histological degree of endobronchial occlusion, the degree of repair, and the degree of granulation, except that these dogs were sacrificed two weeks or four weeks instead of eight weeks after the surgery. Table 3 shows the results.

Evaluation of degree of endobronchial occlusion

The lumen was bronchoscopically observed and evaluated based on the following criteria.

A higher score of the degree of endobronchial occlusion indicates a better endobronchial occlusion effect.

1: The bronchus is occluded.
0: The bronchus is not occluded.

Degree of absorption of material for endobronchial occlusion

The lumen was bronchoscopically observed and evaluated based on the following criteria.

1: The material for endobronchial occlusion is absorbed.
0: The material for endobronchial occlusion is not absorbed.

Presence or absence of displacement

The lumen was bronchoscopically observed and evaluated based on the following criteria.

1: The material for endobronchial occlusion is not displaced.
0: The material for endobronchial occlusion is displaced.

Presence or absence of air leak

The lumen was bronchoscopically observed and evaluated based on the following criteria.

1: No air leak is found.
0: Air leak is found.

Evaluation of occurrence of subcutaneous emphysema

The body surface of each dog was visually observed and evaluated based on the following criteria.

1: No subcutaneous emphysema is found.
0: Subcutaneous emphysema is found.

Evaluation of degree of bronchial repair

The collected tissues were stained with hematoxylin-eosin, and observed with an optical microscope to evaluate the degrees of inflammation, angiogenesis, nerve regeneration, and epithelial regeneration based on the following criteria.

<Inflammation>

3: Mild inflammation
2: Moderate inflammation
1: Severe inflammation

<Angiogenesis>

3: Successful angiogenesis
2: Moderate angiogenesis
1: No growth angiogenesis

<Nerve regeneration>

3: Successful regeneration
2: Moderate regeneration
1: Almost no regeneration

<Epithelial regeneration>

3: Successful regeneration
2: Moderate regeneration
1: Almost no regeneration

Evaluation of degree of granulation

Tissue slices were produced from the collected tissues and stained with hematoxylin-eosin. Then, tissue slice images were obtained with an optical microscope. The tissue slice images were analyzed with ImageJ (National Institutes of Health, USA), and the degree of granulation was evaluated from the following calculation formula.

$$\text{Degree of granulation (\%)} = \text{length of regenerated granulation tissue (mm)}/\text{length of resected bronchi (mm)} \times 100$$

Values of the length of regenerated granulation tissue and the "length of resected bronchi" were obtained by analyzing tissue slice images with ImageJ.

[Table 3]

| | | Material for endobronchial occlusion | Lung resection site | Evaluation results | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|
| | | | | Bronchial occlusion | | | Absorption of material for endobronchial occlusion | | | Displacement | | | Air leak | | | Occurrence of subcutaneous emphysema | | | Histological evaluation | | | | | | | | | | | | Degree of granulation | | |
| | | | | | | | | | | | | | | | | | | | Degree of inflammation | | | Angiogenesis | | | Nerve regeneration | | | Epithelial regeneration | | | | | |
| | | | | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks | 2 weeks | 4 weeks | 8 weeks |
| Evaluaton Example | 1 | Z-1 | Right upper lobe | 1 | 1 | 1 | 0 | 1 | 1 | 1 | 1 | 0 | 0 | 0 | 1 | 1 | 1 | 1 | 3 | 3 | 3 | 1 | 2 | 3 | 1 | 1 | 2 | 1 | 2 | 2 | 75 | 82 | 85 |
| | 2 | Z-2 | Right upper lobe | 1 | 1 | 1 | 0 | 1 | 1 | 1 | 1 | 0 | 0 | 1 | 1 | 1 | 1 | 1 | 2 | 3 | 3 | 2 | 2 | 3 | 1 | 1 | 2 | 1 | 2 | 2 | 88 | 89 | 100 |
| | 3 | Z-3 | Right upper lobe | 1 | 1 | 1 | 0 | 1 | 1 | 1 | 1 | 1 | 1 | 1 | 1 | 1 | 1 | 1 | 2 | 3 | 3 | 2 | 3 | 3 | 2 | 2 | 3 | 2 | 3 | 3 | 96 | 98 | 100 |
| | 4 | Z-4 | Right upper lobe | 1 | 1 | 1 | 0 | 1 | 1 | 1 | 1 | 1 | 1 | 1 | 1 | 1 | 1 | 1 | 2 | 2 | 3 | 1 | 2 | 3 | 1 | 2 | 3 | 1 | 2 | 3 | 97 | 100 | 100 |
| | 5 | Z-5 | Right upper lobe | 1 | 1 | 1 | 0 | 0 | 1 | 1 | 1 | 1 | 1 | 1 | 1 | 1 | 1 | 1 | 2 | 2 | 3 | 1 | 2 | 3 | 1 | 2 | 2 | 1 | 2 | 3 | 75 | 80 | 90 |
| | 6 | Z-6 | Right upper lobe | 1 | 1 | 1 | 0 | 0 | 1 | 1 | 1 | 1 | 1 | 1 | 1 | 1 | 1 | 1 | 2 | 2 | 2 | 1 | 2 | 3 | 1 | 2 | 2 | 1 | 2 | 2 | 65 | 70 | 79 |
| | 7 | Z-4 | Left lower lobe | 1 | 1 | 1 | 0 | 1 | 1 | 1 | 1 | 1 | 1 | 1 | 1 | 1 | 1 | 1 | 2 | 2 | 3 | 1 | 2 | 3 | 1 | 2 | 3 | 1 | 2 | 3 | 96 | 100 | 100 |
| | 8 | Z-7 | Right upper lobe | 1 | 1 | 1 | 0 | 0 | 0 | 1 | 1 | 1 | 1 | 1 | 1 | 1 | 1 | 1 | 1 | 2 | 2 | 1 | 1 | 2 | 1 | 1 | 2 | 1 | 1 | 2 | 75 | 80 | 85 |
| | 9 | Z-8 | Right upper lobe | 1 | 1 | 1 | 0 | 1 | 1 | 1 | 1 | 0 | 0 | 1 | 1 | 1 | 1 | 1 | 2 | 2 | 3 | 1 | 2 | 3 | 1 | 2 | 2 | 1 | 2 | 3 | 85 | 95 | 100 |
| | 10 | Z-9 | Right upper lobe | 1 | 1 | 1 | 0 | 0 | 1 | 1 | 0 | 0 | 0 | 0 | 1 | 1 | 1 | 1 | 2 | 3 | 3 | 1 | 2 | 3 | 1 | 1 | 2 | 1 | 1 | 2 | 75 | 85 | 85 |
| | 11 | Z-10 | Right upper lobe | 1 | 1 | 1 | 0 | 1 | 1 | 1 | 1 | 0 | 1 | 1 | 1 | 1 | 1 | 1 | 2 | 2 | 3 | 1 | 2 | 3 | 1 | 2 | 2 | 1 | 2 | 3 | 75 | 85 | 90 |
| | 12 | Z-11 | Right upper lobe | 1 | 1 | 1 | 0 | 1 | 1 | 1 | 1 | 1 | 1 | 1 | 1 | 1 | 1 | 1 | 1 | 2 | 3 | 1 | 2 | 3 | 1 | 2 | 2 | 1 | 2 | 3 | 65 | 70 | 80 |
| | 13 | Z-12 | Right upper lobe | 1 | 1 | 1 | 0 | 1 | 1 | 1 | 1 | 1 | 1 | 1 | 1 | 1 | 1 | 1 | 2 | 2 | 3 | 1 | 2 | 3 | 1 | 2 | 3 | 1 | 2 | 3 | 90 | 95 | 100 |
| | 14 | Z-13 | Right upper lobe | 1 | 1 | 1 | 0 | 0 | 1 | 1 | 1 | 1 | 1 | 1 | 1 | 1 | 1 | 1 | 1 | 2 | 2 | 1 | 2 | 2 | 1 | 1 | 2 | 1 | 1 | 2 | 55 | 60 | 69 |
| | 15 | Z-14 | Right upper lobe | 1 | 1 | 1 | 0 | 0 | 1 | 1 | 1 | 1 | 1 | 1 | 1 | 1 | 1 | 1 | 1 | 2 | 2 | 1 | 2 | 2 | 1 | 1 | 2 | 1 | 1 | 2 | 55 | 62 | 72 |
| Comparative Evaluation Example | 1 | Z'-1 | Right upper lobe | 0 | 1 | 1 | 1 | 1 | 1 | 0 | 0 | 0 | 0 | 0 | 0 | 1 | 1 | 0 | 2 | 3 | 3 | 1 | 1 | 1 | 1 | 1 | 1 | 1 | 1 | 1 | 50 | 65 | 66 |
| | 2 | Z'-2 | Right upper lobe | 1 | 1 | 1 | 0 | 0 | 0 | 1 | 1 | 1 | 1 | 1 | 1 | 1 | 1 | 1 | 1 | 1 | 2 | 1 | 1 | 1 | 1 | 1 | 1 | 1 | 1 | 1 | 30 | 40 | 50 |
| | 3 | Z'-3 | Right upper lobe | 0 | 0 | 0 | 1 | 1 | 1 | 0 | 0 | 0 | 0 | 0 | 0 | 1 | 1 | 0 | 3 | 3 | 3 | 1 | 1 | 1 | 1 | 1 | 1 | 1 | 1 | 1 | 30 | 40 | 50 |

## INDUSTRIAL APPLICABILITY

The material for endobronchial occlusion of the present invention can be used as a material for endobronchial occlusion to treat air leak from a bronchial stump or a lung stump due to lung resection and promotes tissue repair or replacement. Thus, the material for endobronchial occlusion is suitable as a material for treatment.

SEQUENCE LISTING FREE TEXT

Sequence Listings.txt

## Claims

1. A material for endobronchial occlusion comprising:

a protein (A),
wherein the protein (A) contains at least one of a polypeptide chain (Y) or a polypeptide chain (Y'),
a total number of the polypeptide chain (Y) and the polypeptide chain (Y') in the protein (A) is 1 to 100,
the polypeptide chain (Y) is a polypeptide chain consisting of 2 to 200 tandem repeats of at least one amino acid sequence (X) among an amino acid sequence VPGVG (1) represented by SEQ ID NO: 1, an amino acid sequence GVGVP (4) represented by SEQ ID NO: 4, GPP, GAP, and an amino acid sequence GAHGPAGPK (3) represented by SEQ ID NO: 3,
the polypeptide chain (Y') is a polypeptide chain in which each of a total of 5% or less of amino acids in the polypeptide chain (Y) is replaced by at least one of a lysine residue or an arginine residue, with a total number of the lysine and arginine residues being 1 to 100,
the protein (A) satisfies the following relational expression (1),
the material for endobronchial occlusion has a density of 90 to 450 $mg/cm^3$, and
the material for endobronchial occlusion satisfies the following relational expression (2):

0.50 ≤ [Total number of amino acids constituting the amino acid sequence (X) and amino acid sequence (X') in the protein (A)]/[total number of amino acids constituting the protein (A)] ≤ 0.80 (1),

in the relational expression (1), the amino acid sequence (X') is an amino acid sequence in which each of a total of 60% or less of amino acids in the amino acid sequence (X) is replaced by at least one of a lysine residue or an arginine residue;

$$0.01 \leq Q/P \quad (2)$$

in the relational expression (2), P is the weight of a dried product (DP) of the material for endobronchial occlusion subjected to drying at 100°C and 1 atm for three hours and then drying at 40°C and 1 atm for 15 hours; Q is the weight of a dried product (DQ) of the dried product (DP) subjected to immersion in water having a weight 1000 times the P at 25°C and 1 atm for 72 hours, taking out, drying at 100°C and at 1 atm for three hours, and then drying at 40°C and 1 atm for 15 hours.

2. The material for endobronchial occlusion according to claim 1,

wherein the protein (A) satisfies the following relational expression (3):

$$0.3 \leq R/P \quad (3)$$

in the relational expression (3), P is the weight of a dried product (DP) of the material for endobronchial occlusion subjected to drying at 100°C and 1 atm for three hours and then drying at 40°C and 1 atm for 15 hours; R is the weight of a dried product (DR) of the dried product (DP) subjected to immersion in water having a weight 1000 times the P at 25°C and 1 atm for two hours, taking out, drying at 100°C and 1 atm for three hours, and then drying at 40°C and 1 atm for 15 hours.

**3.** The material for endobronchial occlusion according to claim 1 or 2, wherein the protein (A) contains a polypeptide chain (S) consisting of 2 to 50 tandem repeats of an amino acid sequence GAGAGS (2) represented by SEQ ID NO: 2.

**4.** The material for endobronchial occlusion according to claim 3, wherein a ratio of the number of repeats of GAGAGS (2) to the total number of the amino acid sequence (X) and the amino acid sequence (X') in one molecule of the protein (A), i.e., GAGAGS (2):total number of amino acid sequence (X) and amino acid sequence (X'), is 1:1.5 to 1:20.

**5.** The material for endobronchial occlusion according to any one of claims 1 to 4, wherein the protein (A) is a protein (A11) containing a polypeptide chain (Y'1) in which one amino acid in an amino acid sequence consisting of 2 to 200 tandem repeats of GVGVP (4) is replaced by a lysine residue.

**6.** The material for endobronchial occlusion according to any one of claims 1 to 5, wherein the protein (A) is a protein (A11-2-1) containing a polypeptide chain (S1-1) of $(GAGAGS)_4$ (5) represented by SEQ ID NO: 5, and a polypeptide chain (Y'11) of $(GVGVP)_4$GKGVP $(GVGVP)_3$ (6) represented by SEQ ID NO: 6.

**7.** The material for endobronchial occlusion according to any one of claims 1 to 6, wherein the protein (A) has a molecular mass of 15 to 200 kDa as determined by SDS polyacrylamide gel electrophoresis (SDS-PAGE).

**8.** The material for endobronchial occlusion according to claim 1, wherein the protein (A) contains an amino acid sequence represented by SEQ ID NO: 16, an amino acid sequence represented by SEQ ID NO: 17, an amino acid sequence represented by SEQ ID NO: 20, an amino acid sequence represented by SEQ ID NO: 23, an amino acid sequence represented by SEQ ID NO: 26, an amino acid sequence represented by SEQ ID NO: 27, or an amino acid sequence having a homology of 70% or more with any of these amino acid sequences.

**INTERNATIONAL SEARCH REPORT**

International application No. PCT/JP2020/044316

A. CLASSIFICATION OF SUBJECT MATTER

Int.Cl. C07K5/083(2006.01)i, C07K7/06(2006.01)i, C07K14/00(2006.01)i, A61L31/04(2006.01)i, A61L31/14(2006.01)i
FI: A61L31/04120, A61L31/14, A61L31/14500, C07K14/00, C07K5/083, C07K7/06ZNA

According to International Patent Classification (IPC) or to both national classification and IPC

B. FIELDS SEARCHED

Minimum documentation searched (classification system followed by classification symbols)
Int.Cl. A61L31/04, A61L31/14, C07K5/083, C07K7/06, C07K14/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2016-216453 A (KYOTO UNIVERSITY) 22 December 2016 (2016-12-22), claims 1-3, paragraph [0001], examples 1-12, table 3 | 1-8 |
| Y | JP 2008-125916 A (BIO VERDE KK) 05 June 2008 (2008-06-05), paragraphs [0020], [0021] | 1-8 |
| Y | JP 2004-339395 A (JAPAN ATOMIC ENERGY RESEARCH INSTITUTE) 02 December 2004 (2004-12-02), paragraphs [0014], [0015] | 1-8 |
| Y | JP 2008-001763 A (NATIONAL UNIVERSITY CORPORATION GUNMA UNIVERSITY) 10 January 2008 (2008-01-10), paragraphs [0032], [0033] | 1-8 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 December 2020 | 12 January 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | <br>Telephone No. |

Form PCT/ISA/210 (second sheet) (January 2015)

**INTERNATIONAL SEARCH REPORT**
Information on patent family members

International application No.
PCT/JP2020/044316

| | | |
|---|---|---|
| JP 2016-216453 A | 22 December 2016 | (Family: none) |
| JP 2008-125916 A | 05 June 2008 | (Family: none) |
| JP 2004-339395 A | 02 December 2004 | (Family: none) |
| JP 2008-001763 A | 10 January 2008 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004024864 A
- JP 3338441 B
- JP 4088341 B


### Non-patent literature cited in the description


- Lehninger Principles of Biochemistry. **ALBERT L. LEHNINGER ; DAVID L. NELSON.** Hirokawa Books. September 2010, 346-347
- Seikagaku Jikken Koza 1 (Biochemistry Experimental Course 1), Chemistry of Protein IV. Tokyo Kagakudojin, 01 July 1981
- Sequel to Biochemistry Experimental Course 2. Chemistry of Protein. Tokyo Kagakudojin, 20 May 1987, vol. 2
- **ROBERT F. FADORS et al.** Polymer Engineering and Science. vol. 14, 151-154
- Takehiko Fujimoto. Sanyo Chemical Industries, Ltd, 2007, 212